(19) **Europäisches Patentamt / European Patent Office / Office européen des brevets**

(11) **EP 2 811 979 B1**

(12) **EUROPEAN PATENT SPECIFICATION**

(45) Date of publication and mention of the grant of the patent:
**02.05.2018 Bulletin 2018/18**

(21) Application number: **13706360.8**

(22) Date of filing: **08.02.2013**

(51) Int Cl.:
*A61K 9/08* (2006.01)　　*A61K 47/36* (2006.01)
*A61K 47/02* (2006.01)　　*A61K 47/18* (2017.01)
*A61K 47/34* (2017.01)　　*A61K 47/40* (2006.01)
*A61K 47/44* (2017.01)　　*A61K 31/335* (2006.01)
*A61K 31/551* (2006.01)　　*A61K 9/00* (2006.01)

(86) International application number:
**PCT/US2013/025376**

(87) International publication number:
**WO 2013/119974 (15.08.2013 Gazette 2013/33)**

(54) **AQUEOUS PHARMACEUTICAL COMPOSITION WITH ENHANCED STABILITY**

WÄSSRIGE PHARMAZEUTISCHE ZUSAMMENSETZUNG MIT VERBESSERTER STABILITÄT

COMPOSITION PHARMACEUTIQUE AQUEUSE À STABILITÉ AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2012 US 201261597416 P**

(43) Date of publication of application:
**17.12.2014 Bulletin 2014/51**

(73) Proprietor: **NOVARTIS AG**
**4056 Basel (CH)**

(72) Inventors:
• **MEADOWS, David L.**
**Colleyville**
**Texas 76034 (US)**
• **KETELSON, Howard Allen**
**Dallas**
**Texas 75235 (US)**

• **DAVIS, James W.**
**Suwanee**
**Georgia 30024 (US)**
• **CHIOU, Jang-Shing**
**Arlington**
**Texas 76017 (US)**

(74) Representative: **Stierwald, Michael et al**
**Novartis Pharma AG**
**Patent Department**
**Forum 1**
**Novartis Campus**
**4056 Basel (CH)**

(56) References cited:
**WO-A1-01/54687**　　**WO-A1-03/072081**
**WO-A1-2012/159064**

## Description

### Technical Field of the Invention

The present invention relates to an aqueous pharmaceutical composition, which is preferably at or near physiologic pH and includes an ionized therapeutic agent, an ionic component and guar gum wherein the guar gum limits interactions between the ionized therapeutic agent and the ionic component thereby imparting stability to the composition. More particularly, the present invention relates to an aqueous pharmaceutical composition, preferably an aqueous ophthalmic, otic or nasal composition, which is preferably at or near physiologic pH and which includes a relatively high concentration of ionized therapeutic agent, an ionic component and guar gum wherein the guar gum limits interactions between the ionized therapeutic agent and the ionic component thereby imparting stability to the composition and wherein the composition also preferably further includes a solubizing agent such as a cyclodextrin.

### Background of the Invention

Many pharmaceutical compositions, particularly ophthalmic, otic and nasal compositions, are formed as aqueous compositions (i.e., compositions formed with a significant amount of water) since delivery of these compositions as eyedrops, eardrops, nasal sprays, injections or the like is particularly desirable and aqueous compositions provide a particularly desirable mechanism for such delivery.

One drawback to aqueous composition, however, is that many therapeutic agents exhibit relatively low solubility in water. As such, many aqueous compositions are required to include ingredients such as surfactants, solubilizers or the like to achieve desired concentrations of therapeutic agent in an aqueous composition. However, these ingredients have their own drawbacks. They can be relatively unstable in water. They can irritate ocular tissue as well as other human tissue. Further, they often exhibit difficulty in stabilizing relatively insoluble therapeutic agent within an aqueous composition. Further yet, these ingredients rarely provide any added benefits to the composition other than added solubility. Still further, many of these ingredients must be used at relatively high concentrations to achieve a desired level of solubility and, in turn, can cause other problems such as undesirably high osmolality of an aqueous composition.

As an alternative or in addition to using relatively high concentrations of surfactant, solubilizer or a combination thereof for solubilizing a therapeutic agent, the pH of aqueous compositions can be lowered to enhance solubility of therapeutic agents. Lowering pH of aqueous compositions can, however, be particularly undesirable. In general, human systems tend to maintain a particular pH for aqueous compositions of the body (e.g., mucosa, tear fluid, etc.). This is particularly true of the eye and tear fluid, which is maintained at a pH of approximately 6.8 to 7.2 depending upon the age and health of the eye. When an aqueous composition of relatively low or high pH is dispensed to tear fluid, it can cause several undesirable effects. In particular, upon dispensing, the eye typically quickly begins to tear in an attempt to return to its natural pH. In turn, the introduction of an aqueous composition of relatively high or low pH to the eye can cause stinging and burning sensations in the eye.

These undesirable effects are often enhanced by the nature of the composition being introduced to the eye. For example, in order to maintain a low pH for an ophthalmic composition, the composition must typically include one or more buffers designed to maintain that low pH. When the ophthalmic composition is introduced to the eye, these buffers often enhance the tearing, burning and/or stinging sensations experienced by the eye. This buffering capacity, particularly at low pH, can be particularly difficult for the eye to overcome causing these sensations to linger for undesirable amounts of time.

As an alternative or in addition to using relatively high concentrations of surfactant, solubilizer or a combination thereof and/or lowering pH for increasing therapeutic agent solubility, therapeutic agents are often provided in a manner that allows them to ionize (i.e., have an electronic charge) in an aqueous composition for achieving greater solubility. This mechanism for increasing solubility also suffers from at least one significant drawback. In particular, the ionized therapeutic agent can interact with (e.g., complex with or repel) other ionic components in an aqueous composition and, in turn, undesirably change the dynamics, stability or the like of the composition. This can be particularly detrimental for preserved compositions, which often rely upon a charged preservative for preservation of the composition.

For ophthalmic and/or nasal compositions, antihistamines used to treat allergic conjunctivitis, allergic rhinitis and to potentially aid in treating dry eye have proven particularly difficult to solubilize in aqueous solution. As examples, olopatadine and emedastine are difficult to provide at relatively high concentrations within an aqueous solution and are even more difficult to stabilize at such high concentrations. Since these therapeutic agents are particularly desirable for treating ocular irritation, itchiness, redness and the like and are more effective at relatively high concentrations, it would be particularly desirable to be able to solubilize these agents while avoiding some or all of the aforementioned drawbacks. WO 01/54687 relates to an ophthalmic anti-allergic composition comprising olopatadine HCl or emedastine fumarate and a polymeric quaternary ammonium compound. The composition might further comprise boric acid and viscosity enhancers such as guar gum.

In view of the above, the present invention is directed at a pharmaceutical composition that can provide desirable solubility and/or high concentrations of therapeutic agent within an aqueous composition while avoiding one or more of the drawbacks associated with conventional solubilization techniques. The present invention is additionally or alternatively directed at a pharmaceutical composition that provides a relatively high degree of stability and/or provides retention of the composition on the eye.

## Summary of the Invention

The present invention is directed to an aqueous pharmaceutical composition, according to claim 1. The pharmaceutical composition includes a solubilizing agent, which is a cyclodextrin. The ionized therapeutic agent is olopatadine or emedastine. The ionic component is preferably a preservative such as a polymeric quaternary ammonium compound and benzalkonium chloride (BAC) suitable for preservation of an ophthalmic composition. For ophthalmic use, the composition is preferably disposed in an eyedropper, has a pH of 6.4 to about 7.9, has an osmolality of 200 to 450 or any combination thereof. The composition includes a borate and can also include a polyol. The present invention also relates to the composition according to any of claims 1-8 for use in a method of treating ocular, nasal or otic inflammation, the method comprising topically applying the composition to an eye, an ear or a nose of a human wherein the composition is an otic, ophthalmic or nasal composition. In a preferred embodiment, the step of topically applying the composition includes dispensing an eyedrop of the composition from an eyedropper to the eye.

## Detailed Description of the Invention

The present invention is predicated upon the provision of an aqueous pharmaceutical composition that includes an ionized therapeutic agent, an ionic component and guar gum. The guar gum is present in the composition a concentration sufficient to limit interactions between the ionized therapeutic agent and the ionic component thereby imparting stability to the composition. The guar gum can also be retained on the eye for imparting palliative relief to the eye and/or can aid in retention and penetration of a therapeutic agent upon and into the eye. The composition also typically includes borate for aiding in gelling the guar gum. The composition is preferably at or near physiologic pH. The aqueous pharmaceutical composition has been found particularly useful as an aqueous ophthalmic, otic or nasal composition. The pharmaceutical composition, due to its characteristics is particularly preferred as an ophthalmic composition. The composition will typically include a relatively high concentration of the ionized therapeutic agent. The composition also preferably further includes a solubizing agent such as a cyclodextrin.

Unless indicated otherwise, all component amounts are presented on a % (w/v) basis and all references to therapeutic agent concentration are to concentrations of free base.

The composition typically includes a therapeutic amount of a therapeutic agent. The therapeutic agent is preferably ionized. The ionized therapeutic agent of the composition ionizes within the composition meaning that the therapeutic agent will have an ionic charge when dissolved within the composition. The charge can be negative positive or a combination thereof. The therapeutic agent may ionize by itself within the composition or may ionize as a result of being in salt form upon exposure to the composition.

The therapeutic agent of the composition will also typically be relatively insoluble in water (i.e., will have a relatively low degree of solubility). A therapeutic agent having a relatively low degree of solubility for the present invention means that the therapeutic agent exhibits a solubility in water that is less than 0.01%, more typically less than 0.005%. As used herein, solubility in water is to be determined at 25 °C and atmospheric pressure, unless otherwise specifically stated. These relatively water insoluble therapeutic agents are typically hydrophobic. As such, these agents will typically have a log D that is greater than 0.3, more typically greater than 0.8, more typically greater than 1.5 and even possibly greater than 2.7 or even greater than 5.0.

As used herein, log D is the ratio of the sum of the concentrations of all forms of the therapeutic agent (ionized plus un-ionized) in each of two phases, an octanol phase and a water phase. For measurements of distribution coefficient, the pH of the aqueous phase is buffered to 7.4 such that the pH is not significantly perturbed by the introduction of the compound. The logarithm of the ratio of the sum of concentrations of the solute's various forms in one solvent, to the sum of the concentrations of its forms in the other solvent is called Log D:

$$\log D_{oct/wat} = \log \left( [solute]_{octanol} / \left( [solute]_{ionized\ water} + [solute]_{neutral\ water} \right) \right)$$

The vehicle of the composition of the present invention typically exhibits enhanced ability to solubilize the therapeutic agent relative to water alone. As used herein, the vehicle of the composition is the same as the composition with the exception that the therapeutic agent has been removed or is not added to the composition. For example, the vehicle for an aqueous composition including only 0.5 w/v% olopatadine, 1 w/v% excipient one, 1 w/v% excipient two, 1 w/v% excipient three and water is an aqueous composition including only 1 w/v% excipient one, 1 w/v% excipient two, 1 w/v% excipient three and water. The vehicle of the composition can typically solubilize an amount of therapeutic agent that is at least 110 %, more typically at least 120 % and even more typically at least 130% and even possibly at least 150% by weight of an amount of therapeutic agent that can be solubilized by water. The amounts of therapeutic agent that the vehicle and water can dissolve should be determined at the same conditions discussed herein for solubility in water.

Preferred therapeutic agents are those that alleviate symptoms of allergic conjunctivitis, allergic rhinitis or both. Preferably these agents ionize in the aqueous composition of the present invention and are typically relatively insoluble in water. Antihistaminic agents are particularly desirable. Suitable antihistaminic agents include, without limitation, emedastine, olopatadine, mapinastine, epinastine, levocabastine, loratadine, desloratadine, ketotifen, azelastine, cetirazine, and fexofenadine. Generally, these agents will be added in the form of a pharmaceutically acceptable salt as discussed above. Examples of the pharmaceutically acceptable salts of the antihistaminic agents include inorganic acid salts such as hydrochloride, hydrobromide, sulfate and phosphate; organic acid salts such as acetate, maleate, fumarate (e.g., difumarate), tartrate and citrate; alkali metal salts such as sodium salt and potassium salt; alkaline earth metal salts such as magnesium salt and calcium salt; metal salts such as aluminum salt and zinc salt; and organic amine addition salts such as triethylamine addition salt (also known as tromethamine), morpholine addition salt and piperidine addition salt. Particularly preferred therapeutic agent includes olopatadine HCl, emedastine difumarate or both.

Olopatadine is a known compound that can be obtained by the methods disclosed in U.S. Pat. No. 5,116,863. Olopatadine is an antihistamine (as well as anticholinergic) and mast cell stabilizer. Olopatadine is a selective histamine H1 antagonist. When included, the composition of the present invention contains at least 0.1%, more typically at least 0.33% or 0.55%, even more typically at least 0.65% or 0.67%, still more typically at least 0.7%, but typically no greater than 1.5% more typically no greater than 1.0%, still more typically no greater than 0.8%, possibly no greater than 0.75% and even possibly no greater than 0.72% or 0.6% of olopatadine where concentrations of olopatadine typically represent concentrations of olopatadine in free base form if the olopatadine is added to the composition as a salt. These lower limits of concentrations of olopatadine are particularly important since it has been found that efficacy of olopatadine in aqueous ophthalmic solutions in reducing late phase allergy symptoms begins to show improvement at concentrations greater than 0.5 w/v% of olopatadine and begins to show statistically significant improvements in reducing late phase allergy symptoms at concentrations of about 0.7 w/v% olopatadine and above (e.g., at least 0.65 w/v%, at least 0.67 w/v% or at least 0.68 w/v%). Advantageously, for the composition of the present invention, lower concentrations (e.g., at least 0.50 w/v %, at least 0.55 w/v %, or at least 0.60 w/v %) may show significant improvements and even possibly statistically significant improvements in reducing late phase allergy symptoms.

The most preferred form of olopatadine for use in the solution compositions of the present invention is the hydrochloride salt of (Z)-11-(3-dimethylaminopropylidene)-6,11-dihydro-dibenz-[b,e]oxepin-2-acetic acid. When olopatadine is added to the compositions of the present invention in this salt form, 0.77% olopatadine hydrochloride is equivalent to 0.7% olopatadine free base, 0.88% olopatadine hydrochloride is equivalent to 0.8% olopatadine free base, and 0.99% olopatadine hydrochloride is equivalent to 0.9% olopatadine free base.

When dissolved in the composition, olopatadine is typically substantially entirely or entirely ionized. Olopatadine will have a negative charge due to its carboxylic acid group and a positive charge due to its tertiary amine group.

Emedastine is an H1 antagonist. When included, the composition of the present invention contains at least 0.01%, more typically at least 0.03%, even possibly at least 0.05%, but typically no greater than 0.5% more typically no greater than 0.3%, and even possibly no greater than 0.15% of emedastine where concentrations of emedastine typically represent concentrations of emedastine in free base form if the emedastine is added to the composition as a salt (i.e., as emedastine difumarate).

Guar gum, as used herein, refers to guar gum itself and galactomannans that are derived from guar gum. As used herein, the term "galactomannan" refers to polysaccharides derived from the above natural gums or similar natural or synthetic gums containing mannose or galactose moieties, or both groups, as the main structural components. Preferred guar gums of the present invention are made up of linear chains of (1-4)-β-D-matinopyranosyl units with α-D-galactopyranosyl units attached by (1-6) linkages. With the preferred guar gums, the ratio of D-galactose to D-mannose varies, but generally will be from about 1:2 to 1:4. The guar gum can be substituted in the non-cis hydroxyl positions. An example of non-ionic substitution of guar gum of the present invention is hydroxypropyl guar, with a molar substitution of about 0.4. Molar substitution from 0.01 to 1.2 (e.g. about 0.4) are preferred. Cationic, non-ionic and anionic guar gum or combinations thereof can be used. Anionic substitutions to the guar gum can be particularly preferred when strongly responsive gels are desired. Guar gum is typically present in the composition of the present invention at a concentration of about 0.01 to about 10 w/v %, preferably at about 0.05 w/v % to about 2.0 w/v %, and most preferably at about 0.1 to about 0.5 w/v %. Preferred guar gums of the present invention are guar, hydroxypropyl guar, and hydroxypropyl guar galactomannan. Native guar such as the guar produced by a process set forth in U.S. Patent Application Publication No. 2010/0196415 entitled "Process for Purifying Guar" filed Feb. 5, 2010 is also a preferred guar gum.

As used herein, the term "borate" refers to all pharmaceutically suitable forms of borates, including but not limited to boric acid, organoborates such phenyl boronic acid and alkali metal borates such as sodium borate and potassium borate. Boric acid is the preferred borate used with embodiments of the present invention. Borates are common excipients in ophthalmic formulations due to weak buffering capacity at approximately physiological pH and well known safety and compatibility with a wide range of drugs and preservatives. Borates also have inherent bacteriostatic and fungistatic properties, and therefore aid in the preservation of the compositions. When included, borate is typically present at a concentration of about 0.05 to about 2.0 w/v %, and preferably about 0.1 to 1.5 w/v %.

The ionic component of the present invention can be nearly any chemical entity that exhibits an electrical charge when dissolved in the composition of the present invention. However, in preferred embodiments the ionic component is preservative that exhibits an electrical charge when dissolved in the composition. Examples of preservative include, without limitation, polymeric quaternary ammonium compound, benzalkonium chloride (BAC), polyhexamethylene biguanide (PHMB), alexidine, combinations thereof or the like. Of these preservatives, polymeric quaternary ammonium compound, benzalkonium chloride or a combination thereof are preferred.

The polymeric quaternary ammonium compounds useful in the compositions of the present invention are those which have an antimicrobial effect and which are ophthalmically acceptable. Preferred compounds of this type are described in U.S. Pat. Nos. 3,931,319; 4,027,020; 4,407,791; 4,525,346; 4,836,986; 5,037,647 and 5,300,287; and PCT application WO 91/09523 (Dziabo et al.). The most preferred polymeric ammonium compound is polyquaternium 1, otherwise known as POLYQUAD® or ONAMERM® with a number average molecular weight between 2,000 to 30,000. Preferably, the number average molecular weight is between 3,000 to 14,000.

The polymeric quaternary ammonium compounds, when used, are generally used in the composition of the present invention in an amount that is greater than about 0.0001 w/v %, more typically greater than about 0.0009 w/v % and even more typically greater than about 0.001 w/v % of the composition. Moreover, the polymeric quaternary ammonium compounds are generally used in the composition of the present invention in an amount that is less than about 0.03 w/v %, more typically less than about 0.005 w/v % and even more typically less than about 0.003 w/v % of the composition.

Benzalkonium chloride, when used, is generally used in the composition of the present invention in an amount that is greater than about 0.001 w/v %, more typically greater than about 0.003 w/v % and even more typically greater than about 0.007 w/v % of the composition. Moreover, the polymeric quaternary ammonium compounds are generally used in the compositions of the present invention in an amount that is less than about 0.5 w/v %, more typically less than about 0.05 w/v % and even more typically less than about 0.02 w/v % of the composition.

The composition of the present invention also preferably includes a solubilizing agent, preferably cyclodextrin derivative and more preferably β-cyclodextrin derivative, γ-cyclodextrin derivative or both to aid in solubilizing the therapeutic agent. The β-cyclodextrin derivative, γ-cyclodextrin derivative or combination thereof is typically present in the composition at a concentration that is at least 0.5% w/v, more typically at least 1.0% w/v and even possibly at least 1.3% w/v, but is typically no greater than 6.0% w/v, typically no greater than 4.2% w/v and even possibly no greater than 2.8% w/v.

The specific amount of β-cyclodextrin derivative, γ-cyclodextrin derivative or combination thereof in a particular composition will typically depend upon the type or combination of types of derivatives used. One particularly desirable β-cyclodextrin derivative is a hydroxy alkyl-β-cyclodextrin such as hydroxypropyl-β-cyclodextrin (HP-β-CD). One particularly desirable γ-cyclodextrin derivative is a hydroxy alkyl-γ-cyclodextrin such as hydroxypropyl-γ-cyclodextrin (HP-γ-CD). Another particularly desirable β-cyclodextrin derivative is sulfoalkyl ether-β-cyclodextrin (SAE-β-CD), particularly sulfobutyl ether-β-cyclodextrin (SBE-β-CD). It is contemplated that a combination of hydroxypropyl-β-cyclodextrin, hydroxypropyl-γ-cyclodextrin and/or sulfoalkyl ether-β-cyclodextrin derivative may be employed in a single composition, but it is typically desirable to use only one of the three as the sole or substantially the sole (i.e., at least 90% by weight of the cyclodextrin component) cyclodextrin derivative.

When HP-β-CD is employed as the sole or substantially sole β-cyclodextrin derivative, it is typically present in the composition at a concentration that is at least 0.5% w/v, more typically at least 1.0% w/v and even more typically at least 1.3% w/v, but is typically no greater than 6.0% w/v, typically no greater than 4.2% w/v and is typically no greater than 2.7% w/v. When HP-γ-CD is employed as the sole or substantially sole γ-cyclodextrin derivative, it is typically present in the composition at a concentration that is at least 0.5% w/v, more typically at least 1.0% w/v and even more typically at least 1.3% w/v, but is typically no greater than 6.0% w/v, typically no greater than 4.2% w/v and is typically no greater than 2.7% w/v. When SAE-β-CD is employed as the sole or substantially sole β-cyclodextrin derivative, it is typically present in the composition at a concentration that is at least 0.3% w/v, more typically at least 0.7% w/v and even more typically at least 0.9% w/v, but is typically no greater than 3.4% w/v, typically no greater than 1.9% w/v and is typically no greater than 1.3% w/v.

HP-β-CD is a commodity product and pharmaceutical grades of HP-β-CD can be purchased from a variety of sources, for example, from SIGMA ALDRICH, which has its corporate headquarters in St. Louis, Missouri or ASHLAND SPECIALTY INGREDIENTS, headquartered in Wayne, New Jersey. HP-γ-CD is a commodity product and pharmaceutical grades of HP-γ-CD can be purchased from a variety of sources, for example, from SIGMA ALDRICH, which has its corporate headquarters in St. Louis, Missouri or ASHLAND SPECIALTY INGREDIENTS, headquartered in Wayne, New Jersey. SAE-β-CD can be formed based upon the teachings of U.S. Patent Nos. 5,134,127 and 5,376,645. It is generally preferred, however, to use purified SAE-β-CD. Purified SAE-β-CD is preferably formed in accordance with the teachings of U.S. Patent Nos. 6,153,746 and 7,635,773. Purified SAE-β-CD is commercially available under the tradename CAPTISOL® from CyDex Pharmaceuticals, Inc., Lenexa, KS.

With regard to γ-cyclodextrin derivative and β-cyclodextrin derivative in the composition of the present invention, it has been found that undesirably high concentrations of γ-cyclodextrin derivative and/or β-cyclodextrin derivative can significantly interfere with preservation efficacy of the compositions, particularly when benzalkonium chloride and/or polymeric quaternary ammonium compound are employed as preservation agents. Thus, lower concentrations of γ-cyclodextrin derivative and/or β-cyclodextrin derivative are typically preferred. Advantageously, it has also been found, however, that the ability of the γ-cyclodextrin derivative and β-cyclodextrin derivatives in solubilizing therapeutic agent is very strong and relatively low concentrations of γ-cyclodextrin derivative and/or β-cyclodextrin derivative can solubilize significant concentrations of therapeutic agent in aqueous solution.

The formulations of the present invention may comprise one or more additional excipients. Excipients commonly used in pharmaceutical formulations include, but are not limited to, demulcents, tonicity agents, preservatives, chelating agents, buffering agents, and surfactants. Other excipients comprise solubilizing agents, stabilizing agents, comfort-enhancing agents, polymers, emollients, pH-adjusting agents and/or lubricants. Any of a variety of excipients may be used in formulations of the present invention including water, mixtures of water and water-miscible solvents, such as vegetable oils or mineral oils comprising from 0.5 to 5% non-toxic water-soluble polymers, natural products, such as alginates, pectins, tragacanth, karaya gum, xanthan gum, carrageenin, agar and acacia, starch derivatives, such as starch acetate and hydroxypropyl starch, and also other synthetic products such as polyvinyl alcohol, polyvinylpyrrolidone, polyvinyl methyl ether, polyethylene oxide, and preferably cross-linked polyacrylic acid and mixtures of those products.

Demulcents used with embodiments of the present invention include, but are not limited to, cis diols such as glycerin, propylene glycol and the like, polyvinyl pyrrolidone, polyethylene oxide, polyethylene glycol, and polyacrylic acid. Particularly preferred demulcents are propylene glycol and polyethylene glycol 400.

Suitable tonicity-adjusting agents include, but are not limited to, mannitol, sodium chloride, glycerin, and the like. Suitable buffering agents include, but are not limited to, citrates, phosphates, acetates and the like, and amino alcohols such as 2-amino-2-methyl-1-propanol (AMP). Suitable surfactants include, but are not limited to, ionic and nonionic surfactants (though nonionic surfactants are preferred), RLM 100, POE 20 cetylstearyl ethers such as Procol® CS20, poloxamers such as Pluronic® F68, and block copolymers such as poly(oxyethylene)-poly(oxybutylene) compounds set forth in U.S. Patent Application Publication No. 2008/0138310 entitled "Use of PEO-PBO Block Copolymers in Ophthalmic Compositions" filed Dec. 10, 2007.

Formulations of the present invention are ophthalmically suitable for application to a subject's eyes. The term "aqueous" typically denotes an aqueous formulation wherein the excipient is >50%, more preferably >75% and in particular >90% by weight water. These drops may be delivered from a single dose ampoule which may preferably be sterile and thus render bacteriostatic components of the formulation unnecessary. Alternatively, the drops may be delivered from a multi-dose bottle which may preferably comprise a device which extracts any preservative from the formulation as it is delivered, such devices being known in the art.

The formulations of the present invention are preferably isotonic, or slightly hypotonic in order to combat any hypertonicity of tears caused by evaporation and/or disease. This may require a tonicity agent to bring the osmolality of the formulation to a level at or near 210-320 milliosmoles per kilogram (mOsm/kg). The formulations of the present invention generally have an osmolality in the range of 200 to 400 or 450 mOsm/kg, preferably in the range of 220-320 mOsm/kg, and more preferably in the range of 235-300 mOsm/kg. The ophthalmic formulations will generally be formulated as sterile aqueous solutions.

It is also contemplated that the concentrations of the ingredients comprising the formulations of the present invention can vary. A person of ordinary skill in the art would understand that the concentrations can vary depending on the addition, substitution, and/or subtraction of ingredients in a given formulation.

Preferred formulations are prepared using a buffering system that maintains the formulation at a pH of about 5.5 to about 8.5, more typically about 6.0 to a pH of about 8.3 and more typically about 6.4 to about 7.9. Topical formulations (particularly topical ophthalmic formulations, as noted above) are preferred which have a physiological pH substantially matching the tissue to which the formulation will be applied or dispensed.

It is generally preferred that the composition of the present invention be provided in an eye dropper that is configured to dispense the composition as eyedrops topically to the cornea of the eye.

In a preferred embodiment, the composition of the present invention is a multi-dose ophthalmic compositions that have sufficient antimicrobial activity to allow the compositions to satisfy the USP preservative efficacy requirements, as well as other preservative efficacy standards for aqueous pharmaceutical compositions.

The preservative efficacy standards for multi-dose ophthalmic solutions in the U.S. and other countries/regions are set forth in the following table:

**Preservative Efficacy Test ("PET") Criteria (Log Order Reduction of Microbial Inoculum Over Time**

|  | | Bacteria | Fungi |
|---|---|---|---|
| USP 27 | | A reduction of 1 log (90%), by day 7; 3 logs (99.9%) by day 14; and no increase after day 14 | The compositions must demonstrate over the entire test period, which means no increases of 0.5 logs or greater, relative to the initial inoculum |
| Japan | | 3 logs by 14 days; and no increase from day 14 through day 28 | No increase from initial count at 14 and 28 days |
| Ph. Eur. A[1] | | A reduction of 2 logs (99%) by 6 hours; 3 logs by 24 hours; and no recovery after 28 days | A reduction of 2 logs (99%) by 7 days, and no increase thereafter |
| Ph. Eur. B | | A reduction of 1 log at 24 hours; 3 logs by day 7; and no increase thereafter | A reduction of 1 log (90%) by day 14, and no increase thereafter |
| FDA/ISO 14730 | | A reduction of 3 logs from initial challenge at day 14; and a reduction of 3 logs from rechallenge | No increase higher than the initial value at day 14, and no increase higher than the day 14 rechallenge count through day 28 |
| [1]There are two preservative efficacy standards in the European Pharmacopoeia "A" and "B". | | | |

The standards identified above for the USP 27 are substantially identical to the requirements set forth in prior editions of the USP, particularly USP 24, USP 25 and USP 26.

## Advantages and Problems Overcome

The pharmaceutical composition of the present invention can provide any one or any combination of multiple advantages over pharmaceutical compositions that came before it. As one primary advantage, the guar gum, potentially in the presences of borate, limits the interaction of the charged ionized therapeutic agent with another ionic component (e.g., a charged preservative) thereby enhancing stability of the composition and particularly the stability of the ionic component within the composition. As yet a further additional or alternative advantage, the composition can exhibit enhanced solubility of therapeutic agents, which is particularly desirable for relatively insoluble therapeutic agents. Common ionized therapeutic agents such as olopatadine from olopatadine HCl and emedastine from emedastine difumarate exhibit a synergistic solubility that, without being bound by theory, is believed to be caused by a modification of a guar/borate complex when both guar and borate are present in the composition. Further and without being bound by theory, it is believed that when borate and cyclodextrin are present in the composition, the guar gum is believed to form a charged complex with at least a portion of the cyclodextrin and this complex is believed to be the mechanism for improved stability of relative high concentrations of therapeutic agent. As still another additional or alternative advantage, the guar gum can provide an almost instantaneous palliative relief from ocular inflammatory conditions such as dry eye, allergic conjunctivis or other ocular inflammation providing a level of early relief, which is typically followed by relief provided by the therapeutic agent. As still an even further additional or alternative advantage, the guar gum, due to its viscoelastic properties can aid in maintaining the therapeutic agent upon the ocular surface of the eye for a greater period of time thereby potentially enhancing corneal penetration of the therapeutic agent and/or efficacy of the therapeutic agent through retention on the eye. In turn, this may allow for lower concentrations of therapeutic agents to be used in the composition without any significant loss in efficacy of the therapeutic agent. When an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

Other embodiments of the present invention will be apparent to those skilled in the art from consideration of the present specification and practice of the present invention disclosed herein.

## EXAMPLES

Table A below provides a listing of exemplary ingredients suitable for an exemplary preferred formulation of the pharmaceutical composition of the present invention and a desired weight/volume percentage for those ingredients. It shall be understood that the following Table A is exemplary and that certain ingredients may be added or removed from the Table and concentrations of certain ingredients may be changed while the formulation can remain within the scope of the present invention, unless otherwise specifically stated.

TABLE A

| Ingredient | w/v percent |
|---|---|
| Therapeutic agent (Olopatadine HCl or Emedastine Difuumarate) | 0.5 for Olopatadine HCL 0.05 for Emedastine Difumarate |
| Guar Gum | 0.15 |
| Dibasic Sodium Phosphate (anhydrous) | 0.5 |
| Chelating agent (Disodium EDTA) | 0.005 |
| Borate (Boric Acid) | 0.5 |
| $\gamma$-cyclodextrin derivative and or $\beta$-cyclodextrin derivative | 1.0 for SAE-$\beta$-CD or 1.5 HP-$\beta$-CD or 1.5 HP-$\gamma$-CD |
| Polyol (Mannitol) | 0.3 |
| Polyol (Propylene Glycol) | 1.0 |
| Tonicity Agent (Sodium Chloride) | 0.6 |
| Preservative | 0.01 for BAC or 0.0015 PQAM |
| pH adjusting agents (NaOH or HCl) | sufficient to achieve pH = 7.0 |
| purified water | Q.S. 100 |

It is understood that the weight/volume percents in table A can be varied by $\pm10\%$, $\pm 20\%$, $\pm30\%$, $\pm90\%$ of those weight/volume percents or more and that those variances can be specifically used to create ranges for the ingredients of the present invention. For example, an ingredient weight/volume percent of 10% with a variance of $\pm20\%$ means that the ingredient can have a weight/volume percentage range of 8 to 12 w/v %.

The following examples are presented to further illustrate selected embodiments of the present invention. The formulations shown in the examples were prepared using procedures that are well-known to persons of ordinary skill in the field of ophthalmic pharmaceutical compositions.

**Example 1: Olopatadine in Native Guar Formulation**

This experiment demonstrates the stability of a palliative dry eye / allergy formulations that contains olopatadine and native guar. Three prototype formulations were made and filled in ETO sterilized LDPE opaque DropTainer® (ODT) bottles and clear glass ampules. Their compositions are listed in Table 1-1. Results from stability studies at weeks 0, 3, 6, 15 for olopatadine with native guar solutions A, B and C under various stability conditions (Room Temperature, 40°C, 50°C, 60°C, Freeze/Thaw, Light Cabinet) were monitored. Olopatadine with Native Guar 15 week stability study results and stability trends for all three solutions A, B and C are summarized in Tables 1-2, 1-3.

**Table 1-1.** Formulation Compositions containing Olopatadine and Native Guar

| Formulation Chemical | A w/v% | B w/v% | C w/v% |
|---|---|---|---|
| Olopatadine | 0.111 | 0.111 | 0.111 |
| Purified Native Guar | 0.17 | - | 0.17 |
| Benzalkonium Chloride (BAC) | 0.01 | 0.01 | 0.01 |
| Sodium Chloride | 0.6 | 0.6 | 0.6 |
| Dibasic Sodium Phosphate | 0.5 | 0.5 | 0.5 |
| (Anhydrous) | | | |
| Boric Acid | - | 0.5 | 0.5 |

(continued)

| Formulation Chemical | A w/v% | B w/v% | C w/v% |
|---|---|---|---|
| Purified Water | QS | QS | QS |
| pH | 7.0 | 7.0 | 7.0 |
| Osmolality | 311, 311 | 366, 365 | 390, 388 |

**Table 1-2:** Stability Results Summary for Olopatadine (% Label)

| Formulation | RT | | | | 40 °C | | | 50 °C | | | 60 °C | | F/T | | LC | | |
|---|---|---|---|---|---|---|---|---|---|---|---|---|---|---|---|---|---|
| | Initial | 3 wk | 6 wk | 15 wk | 3 wk | 6 wk | 15 wk | 3 wk | 6 wk | 15 wk | 3 wk | 6 wk | 3 wk | 6 wk | 3 wk | 6 wk | 15 wk |
| A LDPE bottles | 108 | 106 | 106 | 107 | 107 | 106 | 107 | 106 | 108 | 110 | 105 | 108 | 106 | 106 | 106 | 106 | 106 |
| B LDPE bottles | 100 | 99 | 99 | 99 | 99 | 99 | 100 | 100 | 100 | 104 | 101 | 102 | 99 | 99 | 99 | 97 | 99 |
| C LDPE bottles | 106 | 105 | 104 | 106 | 106 | 106 | 108 | 107 | 108 | 110 | 107 | 108 | 105 | 105 | 106 | 105 | 106 |
| A Ampoules | na | na | na | na | na | na | na | na | na | na | na | 104 | na | 105 | na | 106 | 103 |
| B Ampoules | na | na | na | na | na | na | na | na | na | na | na | 98 | na | 98 | na | 99 | 97 |
| C Ampoules | na | na | na | na | na | na | na | na | na | na | na | 104 | na | 109 | na | 105 | 104 |

Table 1-3: Stability Results Summary for Benzalkonium Chloride (% Label)

| BAC (%) | RT | | | | 40°C | | | 50 °C | | | 60 °C | | F/T | | LC | | |
|---|---|---|---|---|---|---|---|---|---|---|---|---|---|---|---|---|---|
| | Initial | 3 wk | 6 wk | 15 wk | 3 wk | 6 wk | 15 wk | 3 wk | 6 wk | 15 wk | 3 wk | 6 wk | 3 wk | 6 wk | 3 wk | 6 wk | 15 wk |
| A LDPE bottles | 102 | 101 | 101 | 105 | 102 | 101 | 107 | 102 | 103 | 109 | 103 | 105 | 101 | 100 | 100 | 102 | 105 |
| B LDPE bottles | 86 | 86 | 86 | 90 | 87 | 86 | 90 | 86 | 88 | 94 | 85 | 90 | 86 | 85 | 86 | 85 | 90 |
| C LDPE bottles | 101 | 101 | 100 | 105 | 101 | 100 | 108 | 102 | 104 | 109 | 102 | 104 | 101 | 101 | 101 | 101 | 105 |
| A Ampoules | na | na | na | na | na | na | na | na | na | na | na | 71 | na | 95 | na | 94 | 73 |
| B Ampoules | na | na | na | na | na | na | na | na | na | na | na | 67 | na | 82 | na | 80 | 68 |
| A Ampoules | na | na | na | na | na | na | na | na | na | na | na | 78 | na | 100 | na | 94 | 80 |

As shown in Table 1-3, 10 to 15 % BAC is lost in formulation B (formulation without native guar). Therefore, native guar aids in formulation stability. Conclusions from the chemical and physical stability results for formulations A and C are as follows:

Chemical stability:

- Olopatadine projected shelf-life is more than 24 months in both containers.
- BAC projected shelf-life is more than 24 months in LDPE-ODT bottle but less than 24 months in glass ampoules;

Physical stability:

- The viscosity is unstable at high temperature (>50°C; for both containers) and therefore preferably stored under light inhibit (LI) conditions.
- The pH and osmolality are stable. Their projected shelf-life is more than 24 months for both containers.
- Visual observation tests of the formulations show that all samples are clear throughout the test period.

The formulations prove to be stable and can be projected to a 2 year shelf-life based on chemical and physical stability.

### Example 2: Emedastine difumarate in HP-Guar formulation

This experiment demonstrates the stability of a palliative dry eye / allergy formulation that contains emedastine and HP-guar. A prototype formulation was made and filled in ETO sterilized clear glass ampoules. The composition is listed in Table 2-1. Results from stability studies at weeks 0, 3, 6, 13, 26 under various stability conditions (RT, 40°C, Freeze/Thaw, Light Cabinet) were monitored. Emedastine with HP-guar of 26 week stability study results and stability trends are summarized in the following Tables 2-2, 2-3.

**Table 2-1:** Formulation Composition containing Emedastine Difumarate and HP-Guar

| Formulation Chemical | (w/v%) |
|---|---|
| Hydroxypropyl Guar | 0.17% |
| Polyquaternium-1 | 0.001% + 10% xs |
| Boric Acid | 0.7% |
| Sorbitol | 1.4% |
| PEG-400 | 0.4% |
| Propylene Glycol | 0.3% |
| Potassium Chloride | 0.12% |
| Sodium Chloride | 0.1% |
| AMP (Ultra) | 0.57% |
| Emedastine Difumarate | 0.05% |
| Hydrochloric Acid | Adjust pH to 7.9 |
| Sodium Hydroxide | Adjust pH to 7.9 |
| Purified Water | QS to 100% |

**Table 2-2:** Stability Results Summary for HP-Guar / Emedastine Difumarate Formulation Stored at Room Temperature and 40°C for 26 Weeks

| | Initial | Room Temperature | | | | | 40°C | | | | |
| --- | --- | --- | --- | --- | --- | --- | --- | --- | --- | --- | --- |
| | Wk 0 | Wk 2 | Wk 4 | Wk 6 | Wk 13 | Wk 26 | Wk 2 | Wk 4 | Wk 6 | Wk 13 | Wk 26 |
| Polyquad (ppm) | 9.9 | 10.2 | 10.1 | 10.2 | 9.7 | 9.6 | 10.0 | 10.0 | 10.2 | 8.0 | 5.3 |
| Emedastine ( % Label) | 99 | 99 | 97 | 97 | 100 | 96 | 98 | 97 | 97 | 97 | 95 |
| pH | 7.7 | 7.7 | 7.7 | 7.7 | 7.7 | 7.8 | 7.7 | 7.7 | 7.7 | 7.7 | 7.8 |
| Osmolality (mOsm/kg) | 291 | 292 | 288 | 289 | 288 | 289 | 289 | 290 | 290 | 286 | 291 |
| Viscosity (cPs) | 13 | 13 | 13 | 12 | 12 | 12 | 12 | 12 | 12 | 11 | 9 |

**Table 2-3:** Stability Results Summary for HP-Guar / Emedastine Difumarate Formulation under Freeze/Thaw Conditions for 6 Weeks and Light Cabinet Conditions for 13 Weeks

| Batch Record LN 14239:99-100 | Initial | Freeze/Thaw | | | Light Cabinet | | | |
| --- | --- | --- | --- | --- | --- | --- | --- | --- |
| | Wk 0 | Wk 2 | Wk 4 | Wk 6 | Wk2 | Wk 4 | Wk 6 | Wk 13 |
| Polyquad (ppm) | 9.9 | 10.02 | 10.4 | 10.2 | 9.9 | 9.9 | 9.7 | 9.0 |
| Emedastine (% Label) | 99 | 98.28 | 97.5 | 98.5 | 95.5 | 91.0 | 87.6 | 75.7 |
| pH | 7.7 | 7.7 | 7.7 | 7.7 | 7.7 | 7.7 | 7.7 | 7.7 |
| Osmolality (mOsm/kg) | 291 | 290 | 292 | 291 | 294 | 289 | 294 | 284 |
| Viscosity (cPs) | 13 | 12.9 | 13.3 | 13.0 | 11.0 | 9.5 | 8.4 | 5.5 |

**Conclusion:**

Conclusions from the chemical and physical stability results are as follows:

Chemical stability:

- Emedastine difumarate projected shelf-life is more than 24 Months
- PQ projected shelf-life is more than 24 Months (at room temperature (RT)) under light inhibit (LI) conditions; less than 24 Months (at high temperature);

Physical stability:

- The viscosity is unstable at high temperature (>40°C) and therefore preferably stored under light inhibit (LI) conditions.
- The pH and osmolality are stable. The projected shelf-life is more than 24 Months.
- Visual observation tests of the formulations show that all samples are clear throughout the test period.

The formulation proves to be stable and can be projected to 2 year shelf-life based on chemical and physical stability at room temperature.

**Example 3 High Dose Olopatadine Solubility in Native Guar with additional excipients**

These experiments demonstrate the solubility of 0.5% to 1% olopatadine in native guar formulations with additional excipients, Hydroxypropyl-Beta-Cyclodextrin (HPCD). Twenty prototype formulations were made to evaluate the solubility range from 0.111% to 1%. Various concentrations of HPCD were used in the formulations. The formulations were filled in glass scintillation vials and in LDPE Drop-Tainers. Their compositions are listed in Table 3-1 to Table 3-3. The formulations were evaluated for clarity using UV light.

**Example 4**

**Table 4-1:** Formulation Compositions Containing 1.0% Olopatadine in 0.17% Native Guar and 1% to 6% Hydroxypropyl-Beta-Cyclodextrin (HPCD).

| Formulation Chemical | A w/v% | B w/v% | C w/v% | D w/v% | E w/v% |
|---|---|---|---|---|---|
| Olopatadine | 1.0 | 1.0 | 1.0 | 1.0 | 1.0 |
| HPCD | - | 1.0 | 3.0 | 5.0 | 6.0 |
| Purified Guar | 0.17 | 0.17 | 0.17 | 0.17 | 0.17 |
| Sorbitol | 1.0 | 1.0 | 1.0 | 1.0 | 1.0 |
| Sodium Borate, decahydrate | 0.5 | 0.5 | 0.5 | 0. 5 | 0.5 |
| Boric Acid | 0.5 | 0.5 | 0.5 | 0.5 | 0.5 |
| Sodium Phosphate, Dibasic | 0.5 | 0.5 | 0.5 | 0.5 | 0.5 |
| Sodium Citrate | 0.35 | 0.35 | 0.35 | 0.35 | 0.35 |
| Sodium Chloride | 0.15 | 0.15 | 0.15 | 0.15 | 0.15 |
| Purified Water | QS | QS | QS | QS | QS |
| Target pH | 7.0 | 7.0 | 7.0 | 7.0 | 7.0 |
| : Actual pH | 7.04 | 7.06 | 7.04 | 7.01 | 7.02 |
| Physical Observation | Cloudy Precipitate | Cloudy Precipitate | Cloudy Precipitate | clear | clear |
| Physical Observation a/f 1st FT | - | - | - | clear | clear |
| pH a/f 1st FT | - | - | - | 7.01 | 7.02 |
| Physical Observation a/f 2nd FT | - | - | - | clear | clear |
| pH a/f 2nd FT | - | - | - | 6.99 | 6.98 |
| Physical Observation a/f 3rd FT-wkd freeze | - | - | - | clear | clear |
| pH a/f a/f 3rd FT- wkd freeze | - | - | - | 7.00 | 7.02 |
| Physical Observation a/f 4th FT | - | - | - | clear | clear |
| Physical Observation a/f 5th FT | - | - | - | clear | clear |
| pH a/f a/f 5th FT | - | - | - | 7.04 | 7.03 |

(continued)

| Formulation Chemical | A w/v% | B w/v% | C w/v% | D w/v% | E w/v% |
|---|---|---|---|---|---|
| Physical " Observation a/f 6th FT - 5day freeze | - | - | - | clear | clear |

**Conclusion:**

- 1% Olopatadine formulation precipitates out below 3% HPCD for these particular formulations.
- With 0.17% of native guar and approximately 5% or more HPCD, Olopatadine at a concentration of 1% is solubilized at physiologic pH of approximately 7.
- Formulations (15528-81D and 15528-81E) are clear and the pH are stable at pH 7.0 after 5 freeze/thaw from -20°C to room temperature cycle.

**Example 5**

**Table 5-1:** Formulation Compositions Containing 0.5% and 1.0% Olopatadine in 0.17% Native Guar and 1% to 6% Hydroxypropyl-Beta-Cyclodextrin (HPCD). This experiment optimizes the solubility by combination of Guar and cyclodextrin.

**Example 5**

**Table 5-1:** Formulation Compositions Containing 0.5% and 1.0% Olopatadine in 0.17% Native Guar and 1% to 6% Hydroxypropyl-Beta-Cyclodextrin (HPCD).

| Formulation Chemical | A w/v% | B w/v% | C w/v% | D w/v% | E w/v% | F w/v% | G w/v% | H w/v% |
|---|---|---|---|---|---|---|---|---|
| Olopatadine | 1.0 | 1.0 | 1.0 | 1.0 | 1.0 | 0.5 | 0.5 | 0.5 |
| HPCD | - | 3.0 | 4.0 | 5.0 | 6.0 | 0.5 | 1.0 | 2.0 |
| Purified Guar | 0.17 | 0.17 | 0.17 | 0.17 | 0.17 | 0.17 | 0.17 | 0.17 |
| Sorbitol | 1.0 | 1.0 | 1.0 | 1.0 | 1.0 | 1.0 | 1.0 | 1.0 |
| Sodium Borate, decahydrate | 0.5 | 0.5 | 0.5 | 0.5 | 0.5 | 0.5 | 0.5 | 0.5 |
| Boric Acid | 0.17 | 0.17 | 0.17 | 0.17 | 0.17 | 0.17 | 0.17 | 0.17 |
| Sodium Phosphate, Dibasic | 0.5 | 0.5 | 0.5 | 0.5 | 0.5 | 0.5 | 0.5 | 0.5 |
| Sodium Citrate | 0.35 | 0.35 | 0.35 | 0.35 | 0.35 | 0.35 | 0.35 | 0.35 |
| Sodium Chloride | 0.15 | 0.15 | 0.15 | 0.15 | 0.15 | 0.35 | 0.15 | 0.15 |
| BAC | 0.01 | 0.01 | 0.01 | 0.01 | 0.01 | 0.01 | 0.01 | 0.01 |
| Purified Water | QS | QS | QS | QS | QS | QS | QS | QS |
| Target pH | 7.0 | 7.0 | 7.0 | 7.0 | 7.0 | 7.0 | 7.0 | 7.0 |
| Actual pH | 7.05 | 7.01 | 7.03 | 7.03 | 7.00 | 7.03 | 6.98 | 6.99 |
| Physical observation b/f FT | Cloudy precipitate | clear | clear | clear | clear | clear | clear | clear |
| Physical observation a/f 1st FT (freeze thaw) | - | clear | clear | clear | clear | clear | clear | clear |

(continued)

| Formulation Chemical | A w/v% | B w/v% | C w/v% | D w/v% | E w/v% | F w/v% | G w/v% | H w/v% |
|---|---|---|---|---|---|---|---|---|
| pH a/f 1st FT | - | 7.06 | 7.07 | 6.99 | - | - | - | - |
| Physical observation a/f 2nd FT | - | clear | clear | clear | clear | clear | clear | clear |
| Physical observation a/f 3rd FT | - | Cloudy precipitate | clear | clear | clear | Cloudy precipitate | Cloudy precipitate | clear |
| pH a/f 3rd FT | - | 7.05 | 7.06 | 6.99 | 7.04 | 7.08 | 7.03 | 7.02 |
| Physical observation a/f 4th FT-5 day cycle | - | cloudy precipitate | clear | clear | clear | Cloudy precipitate | Cloudy precipitate | clear |

**Conclusion:**

- 0.5% and 1% Olopatadine formulations precipitated out below approximately 1% and 3% HPCD after 2nd or 3rd Freeze-Thaw cycles respectively.
- With 0.17% of native guar and increase HPCD to at or above approximately 2% or 4% in the Olopatadine formulations, the solubility increases to 0.5% and 1% at physiologic pH of 7.
- Formulations (C, D, E, and H) are clear and the pH are stable at 7.0 after 4 freeze/thaw from -20°C to room temperature cycle.
- A concentration of cyclodextrin at physiologic pH of 7 that can solubilize 0.5% and 0.6% Olopatadine together with 0.17% guar are approximately 2% and 4% cyclodextrin respectively.

**Example 6**

**Table 6-1:** Formulation Compositions Containing 0.7% Olopatadine in 0.17% Native Guar and 5.0% Hydroxypropyl-Beta-Cyclodextrin (HPCD). This experiment shows 16 weeks stability of Guar/HPCD/BAC present in 0.7% Olopatadine formulations.

| Formulation Chemical | w/v% |
|---|---|
| Olopatadine | 0.7 |
| Purified Guar | 0.17 |
| HPCD | 5 |
| Benzalkonium Chloride | 0.01 |
| Sorbitol | 1 |
| Sodium Chloride | 0.1 1 |
| Sodium Borate (Decahydrate) | 0.5 |
| Sodium Citrade (Dihydrate) | 0.35 |
| Dibasic Sodium Phosphate (Anhydrous) | 0.1 |
| Boric Acid | 0.17 |
| Purified Water | QS |
| pH | 7.0 |

(continued)

| Formulation Chemical | w/v% |
|---|---|
| Osmolality | 298 |

Table 5-2. AL04943A with Purified Guar/HPCD FID 117941 16 week stability study result summary

| Olopatadine with Purified Guar/HPCD -1 - 16 Week Result Summary | | | | | | | | | | | | |
|---|---|---|---|---|---|---|---|---|---|---|---|---|
| | RT | | | | 40 °C | | | 50 °C | | | 60°C | F/T | LC |
| | Initial | 3 wk | 6 wk | 16 wk | 3 wk | 6 wk | 16 wk | 3 wk | 6 wk | 16 wk | 6 wk | 6 wk | 6 wk |
| Olopatadine (ppm) | 6451 | 6475 | 6507 | 6405 | 6557 | 6513 | 6571 | 7040 | 6603 | 6912 | 6753 | 6484 | 6502 |
| BAC (ppm) | 85.9 | 83.5 | 83.9 | 84.4 | 83.6 | 83.9 | 86.0 | 84.7 | 85.6 | 89.7 | 87.5 | 83.6 | 84.0 |
| pH | 7.03 | 7.03 | 7.02 | 7.03 | 7.03 | 6.99 | 6.99 | 7.00 | 6.98 | 6.96 | 6.95 | 7.02 | 6.93 |
| Osmolality (mOsm/kg) | 298 | 298 | 300 | 293 | 299 | 302 | 308 | 303 | 308 | 326 | 325 | 298 | 307 |
| Viscosity (cPs) | 7.81 | 8.01 | 8.01 | 8.09 | 7.86 | 7.83 | 7.91 | 7.69 | 7.51 | 7.46 | 6.82 | 8.03 | 7.59 |
| Weigh Loss (%) | 0.00 | 0.04 | 0.08 | 0.22 | 0.23 | 0.49 | 1.41 | 0.67 | 1.34 | 3.47 | 2.54 | 0.03 | 0.14 |
| Physical Observation | Clear | Clear | Clear | Clear | Clear | Clear | Clear | Clear | Clear | Clear | Clear | Clear | Clear |

**Conclusion:**

- 0.7% olopatadine formulations are clear with 5% HPCD and 0.17% of native guar in the olopatadine formulations at physiologic pH of 7 after 16 weeks at RT, 40 °C, 50 °C and 6 weeks after 60 °C, light cabinet, and 3 Freeze-Thaw (-18 °C to RT) cycles conditions.
- Physical (Osmolality, viscosity, pH, clear) and chemical (Olopatadine and BAC) of the formulation are stable during the 16 weeks stability study, which can projected 2 years shelf-lift stability from this accelerated study.

**Claims**

1.  An aqueous pharmaceutical composition, comprising:

    an ionized therapeutic agent selected from the group consisting of olopatadine and emedastine;
    an ionic component;
    guar gum at a concentrations sufficient to limit interactions between the ionized therapeutic agent and the ionic component thereby imparting stability to the composition;
    a cyclodextrin;
    borate; and
    water;
    wherein the composition has a pH that is at least 6.0, but is no greater than 8.3;
    with the proviso that the following formulation is excluded:

    | Ingredients | % (w/v) |
    | --- | --- |
    | Olopatadine HCL | 0.77 |
    | Dibasic Sodium Phosphate, anhydrous | 0.1 |
    | Purified Guar | 0.17 |
    | Hydroxypropyl-$\beta$-Cyclodextrin | 5 |
    | Boric Acid | 0.17 |
    | Sodium Borate, decahydrate | 0.5 |
    | Sodium Chloride | 0.1 |
    | Sorbitol | 1 |
    | Sodium Citrate, dihydrate | 0.35 |
    | Benzalkonium Chloride | 0.01 |
    | Sodium Hydroxide / Hydrochloric Acid | q.s. to pH 7.0 |
    | Purified water | q.s. to 100% |

2.  The pharmaceutical composition of claim 1 wherein the olopatadine is olopatadine HCl and the emedastine is emedastine difumarate.

3.  The pharmaceutical composition of any of the preceding claims wherein the ionic component is a preservative.

4.  The pharmaceutical composition of claim 3 wherein the preservative is selected from the group consisting of a polymeric quaternary ammonium compound and benzalkonium chloride.

5.  The pharmaceutical composition of any of the preceding claims wherein the guar gum is selected from the group consisting of native guar and hydroxypropyl guar.

6.  The pharmaceutical composition of any of the preceding claims wherein the pharmaceutical composition is formulated as an ophthalmic composition suitable for topical application to the eye.

7.  The pharmaceutical composition of any of the preceding claims wherein the composition is disposed in an eyedropper, has a pH of about 6.4 to about 7.9, has an osmolality of 200 to 450 or any combination thereof.

8.  The pharmaceutical composition as in any of the preceding claims further comprising polyol.

9.  The composition according to any of the preceding claims for use in a method of treating ocular, nasal or otic inflammation, the method comprising topically applying the composition to an eye, an ear or a nose of a human wherein the composition is an otic, ophthalmic or nasal composition.

10. The composition for use according to claim 9, wherein the composition is an ophthalmic composition and the step of topically applying the composition includes dispensing an eyedrop from an eyedropper to the eye.

**Patentansprüche**

1. Wässrige pharmazeutische Zusammensetzung, umfassend:

   ein ionisiertes Therapeutikum ausgewählt aus der Gruppe bestehend aus Olopatadin und Emedastin,
   eine ionische Komponente,
   Guargummi in einer Konzentration, die ausreicht, um die Wechselwirkungen zwischen dem ionisierten Therapeutikum und der ionischen Komponente zu begrenzen und dadurch der Zusammensetzung Stabilität zu verleihen,
   ein Cyclodextrin,
   Borat und
   Wasser,
   wobei die Zusammensetzung einen pH-Wert von mindestens 6,0, jedoch nicht mehr als 8,3, aufweist,
   mit der Maßgabe, dass die folgende Formulierung ausgeschlossen ist:

   | Inhaltsstoffe | % (w/v) |
   |---|---|
   | Olopatadin-HCl | 0,77 |
   | Dinatriumhydrogenphosphat, wasserfrei | 0,1 |
   | Gereinigtes Guargummi | 0,17 |
   | Hydroxypropyl-β-cyclodextrin | 5 |
   | Borsäure | 0,17 |
   | Natriumboart-decahydrat | 0,5 |
   | Natriumchlorid | 0,1 |
   | Sorbitol | 1 |
   | Natriumcitrat-dihydrat | 0,35 |
   | Benzalkoniumchlorid | 0,01 |
   | Natriumhydroxid/Salzsäure | q.s. bis zu einem pH-Wert von 7,0 |
   | Gereinigtes Wasser | q.s. bis 100% |

2. Pharmazeutische Zusammensetzung nach Anspruch 1, wobei es sich bei dem Olopatadin um Olopatadin-HCl handelt und es sich bei dem Emedastin um Emedastindifumarat handelt.

3. Pharmazeutische Zusammensetzung nach einem der vorhergehenden Ansprüche, wobei es sich bei der ionischen Komponente um einen Konservierungsstoff handelt.

4. Pharmazeutische Zusammensetzung nach Anspruch 3, wobei der Konservierungsstoff aus der aus einer polymeren quartären Ammoniumverbindung und Benzalkoniumchlorid bestehenden Gruppe ausgewählt ist.

5. Pharmazeutische Zusammensetzung nach einem der vorhergehenden Ansprüche, wobei das Guargummi aus der aus nativem Guargummi und Hydroxypropylguar bestehenden Gruppe ausgewählt ist.

6. Pharmazeutische Zusammensetzung nach einem der vorhergehenden Ansprüche, wobei die pharmazeutische Zusammensetzung als eine für die topische Anwendung am Auge geeignete ophthalmische Zusammensetzung formuliert ist.

7. Pharmazeutische Zusammensetzung nach einem der vorhergehenden Ansprüche, wobei die Zusammensetzung sich in einer Augentropfenpipette befindet, einen pH-Wert von etwa 6,4 bis etwa 7,9 aufweist, eine Osmolalität von 200 bis 450 aufweist oder eine beliebige Kombination davon aufweist.

8. Pharmazeutische Zusammensetzung nach einem der vorhergehenden Ansprüche, welche weiterhin ein Polyol umfasst.

9. Zusammensetzung nach einem der vorhergehenden Ansprüche zur Verwendung in einem Verfahren zur Behandlung einer Augen-, Nasen- oder Ohrentzündung, wobei das Verfahren die topische Anwendung der Zusammensetzung am Auge, am Ohr bzw. an der Nase eines Menschen umfasst, wobei es sich bei der Zusammensetzung um eine otische, ophthalmische oder nasale Zusammensetzung handelt.

10. Zusammensetzung zur Verwendung nach Anspruch 9, wobei es sich bei der Zusammensetzung um eine ophthalmische Zusammensetzung handelt und der Schritt der topischen Anwendung der Zusammensetzung die Abgabe eines Augentropfens aus einer Augentropfenpipette an das Auge einschließt.

## Revendications

1. Composition pharmaceutique aqueuse, comprenant :

   un agent thérapeutique ionisé choisi dans le groupe constitué de l'olopatadine et l'émédastine ;
   un composant ionique ;
   de la gomme de guar à une concentration suffisante pour limiter les interactions entre l'agent thérapeutique ionisé et le composant ionique de façon à conférer une stabilité à la composition ;
   une cyclodextrine ;
   du borate ; et
   de l'eau ;
   la composition ayant un pH qui est d'au moins 6,0, mais n'est pas supérieur à 8,3 ;
   à condition que la formulation suivante soit exclue :

   | Composants | % (m/v) |
   |---|---|
   | Olopatadine HCl | 0,77 |
   | Phosphate de sodium dibasique, anhydre | 0,1 |
   | Guar purifié | 0,17 |
   | Hydroxypropyl-$\beta$-cyclodextrine | 5 |
   | Acide borique | 0,17 |
   | Borate de sodium, décahydraté | 0,5 |
   | Chlorure de sodium | 0,1 |
   | Sorbitol | 1 |
   | Citrate de sodium, dihydraté | 0,35 |
   | Chlorure de benzalkonium | 0,01 |
   | Hydroxyde de sodium / acide chlorhydrique | q.s. pour pH 7,0 |
   | Eau purifiée | q.s. pour 100 % |

2. Composition pharmaceutique de la revendication 1 dans laquelle l'olopatadine est l'olopatadine HCl et l'émédastine est le difumarate d'émédastine.

3. Composition pharmaceutique de l'une quelconque des revendications précédentes dans laquelle le composant ionique est un conservateur.

4. Composition pharmaceutique de la revendication 3 dans laquelle le conservateur est choisi dans le groupe constitué d'un composé d'ammonium quaternaire polymère et du chlorure de benzalkonium.

5. Composition pharmaceutique de l'une quelconque des revendications précédentes dans laquelle la gomme de guar est choisie dans le groupe constitué de guar natif et d'hydroxypropyl-guar.

6. Composition pharmaceutique de l'une quelconque des revendications précédentes, la composition pharmaceutique étant formulée sous la forme d'une composition ophtalmique adaptée pour application topique sur l'oeil.

7. Composition pharmaceutique de l'une quelconque des revendications précédentes, la composition étant disposée dans un compte-gouttes oculaire, ayant un pH d'environ 6,4 à environ 7,9, ayant une osmolalité de 200 à 450 ou une combinaison quelconque de ceux-ci.

8. Composition pharmaceutique selon l'une quelconque des revendications précédentes comprenant en outre un polyol.

9. Composition selon l'une quelconque des revendications précédentes pour utilisation dans un procédé de traitement d'une inflammation oculaire, nasale ou otique, le procédé comprenant l'application topique de la composition sur un oeil, une oreille ou un nez d'un humain, la composition étant une composition otique, ophtalmique ou nasale.

10. Composition pour utilisation selon la revendication 9, la composition étant une composition ophtalmique et l'étape d'application topique de la composition comprenant la distribution d'une goutte oculaire depuis un compte-gouttes oculaire sur l'oeil.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0154687 A
- US 5116863 A
- US 20100196415 A
- US 3931319 A
- US 4027020 A
- US 4407791 A
- US 4525346 A
- US 4836986 A
- US 5037647 A
- US 5300287 A
- WO 9109523 A, Dziabo
- US 5134127 A
- US 5376645 A
- US 6153746 A
- US 7635773 B
- US 20080138310 A